# EUROPÄISCHE PATENTSCHRIFT

(11) **EP 3 082 559 B1**
(45) Veröffentlichungstag und Bekanntmachung des Hinweises auf die Patenterteilung: **17.07.2019**
(21) Anmeldenummer: 14821572.6
(22) Anmeldetag: 16.12.2014
(51) Int. Cl.: A61B 1/00, A61B 1/005, F16C 1/10, F16C 1/26, A61M 25/00, B60T 11/04, B29C 45/00, B29C 45/03

(54) **VORRICHTUNG UND VERFAHREN ZUM HERSTELLEN EINES LÄNGLICHEN HOHLPROFILELEMENTS**
DEVICE AND METHOD FOR PRODUCING AN ELONGATED HOLLOW PROFILE ELEMENT
DISPOSITIF ET PROCÉDÉ DE PRODUCTION D'UN ÉLÉMENT PROFILÉ CREUX ALLONGÉ

(30) Priorität: 19.12.2013 DE 102013226591
(43) Veröffentlichungstag der Anmeldung: 26.10.2016
(73) Patentinhaber: Digital Endoscopy GmbH, 86316 Friedberg (DE)
(72) Erfinder: VIEBACH, Thomas, 86579 Waidhofen (DE); PAUKER, Fritz, 86420 Diedorf (DE)
(74) Vertreter: TBK
(86) Internationale Anmeldenummer: PCT/EP2014/077938
(87) Internationale Veröffentlichungsnummer: WO 2015/091464

(56) Entgegenhaltungen:
- EP-A1- 1 475 031
- DE-A1-102010 034 623
- JP-A- H06 254 049
- US-A1- 2006 199 999
- US-A1- 2011 288 372

## Beschreibung

Die vorliegende Erfindung betrifft eine Vorrichtung und ein Verfahren zum Herstellen eines länglichen Hohlprofilelements, ein längliches Hohlprofilelement und eine Abwinkelungseinheit für ein Endoskop. Insbesondere betrifft die vorliegende Erfindung ein solches längliches Hohlprofilelement, das biegbar ist und ein sich durch dieses erstreckendes Innenelement umgibt.

Ein solches längliches Hohlprofilelement findet seinen Einsatz beispielsweise als Drahtführung für eine Fahrradbremse oder Fahrradgangschaltung. Darüber hinaus werden solche länglichen Hohlprofilelemente in einer Abwinkelungseinheit für ein Endoskop angewendet.

Bei einem Endoskop wird nämlich ein biegbares Ende eines Katheters, das heißt ein sogenannter Deflectingabschnitt als Abwinkelungseinheit, durch Schwenken eines Steuerelementes bewegt, das z.B. Zugseilelemente betätigt, die mit dem Kopf des Deflectingabschnittes verbunden sind. Dadurch soll die Bewegung des Deflectingabschnittes genau der Bewegung des Steuerelementes folgen. In diesem Deflectingabschnitt umgibt ein längliches Hohlprofilelement die Innenelemente des Deflectingabschnittes, wie z.B. die Zugseilelemente, einen Arbeitskanal, etc.

Bei medizinischen Untersuchungen mit einem Endoskop sollte die Übertragung einer Schwenkbewegung eines Steuerelementes zu einer Biegebewegung des Deflectingabschnittes möglichst genau sein. Andererseits sollte die Übertragung einer Schwenkbewegung eines Steuerelementes zu einer Biegebewegung des Deflectingabschnittes für den Anwender einfach und übersichtlich ausführbar sein.

### DURCH DIE ERFINDUNG ZU LÖSENDE AUFGABE

Aufgabe der vorliegenden Erfindung ist es, eine verbesserte Vorrichtung und ein verbessertes Verfahren zum Herstellen eines länglichen Hohlprofilelements zu schaffen. Ausserdem sollen ein längliches Hohlprofilelement und eine Abwinkelungseinheit für ein Endoskop geschaffen werden.

### LÖSUNG DER AUFGABE

In Hinblick auf die Vorrichtung ist diese Aufgabe erfindungsgemäß durch eine Vorrichtung mit den Merkmalen von Anspruch 1 gelöst.

In Hinblick auf das Verfahren ist diese Aufgabe erfindungsgemäß durch ein Verfahren mit den Merkmalen von Anspruch 15 gelöst.

Vorteilhafte Weiterbildungen sind Gegenstand der abhängigen Ansprüche.

Die Erfindung betrifft somit eine Vorrichtung zum Herstellen eines länglichen Hohlprofilelements, das zumindest einen Zugdraht aufweist, mit: zwei an entgegengesetzten Seiten der Vorrichtung angeordneten Gegenlagern, die den Längsenden des länglichen Hohlprofilelements entsprechen, wobei am distalen Gegenlager ein Ende des zumindest einen Zugdrahtes verankert ist, und einer Form, die einen mit einem aushärtenden Material befüllbaren abgedichteten länglichen Hohlraum ausbildet, wobei der Hohlraum sich zwischen den beiden Gegenlagern erstreckt und im Hohlraum der Zugdraht so verläuft, dass er die Hohlraumwand nicht berührt.

In der Vorrichtung können die Gegenlager so beabstandet sein, dass der Zugdraht gespannt ist. Dadurch können der Zugdraht oder die Zugdrähte sich vordefiniert in gerader Richtung zwischen den Gegenlagern erstrecken. Nach dem Aushärten des einzufüllenden Materials sind somit Zugdrahtkanäle vorhanden, die gerade verlaufen. In den Zugdrahtkanälen sind bereits die Zugdrähte angeordnet.

In der Vorrichtung kann außer dem zumindest einen Zugdraht im Hohlraum zumindest ein Schlauchelement zur Ausbildung eines Arbeitskanals so angeordnet sein, dass die Enden des zumindest einen Schlauchelementes jeweils in den Gegenlagern verankert sind.

Alternativ kann in der Vorrichtung außer dem zumindest einen Zugdraht im Hohlraum zumindest ein entnehmbares Dornelement zur Ausbildung eines Arbeitskanals so angeordnet sein, dass die Enden des zumindest einen Dornelementes jeweils in den Gegenlagern sitzen.

In der Vorrichtung kann das Dornelement am distalen Ende einen aussermittigen Endabschnitt aufweisen. Das Dornelement kann also so ausgebildet sein, dass es einen proximalen Endabschnitt, der in bezug auf die Form und den Hohlraum mittig d.h. auf der Längsachse/Mittelachse der Form und des Hohlraums, die zueinander koaxial sein können, ist, und einen distalen Endabschnitt hat, der in bezug auf die Form und den Hohlraum aussermittig d.h. nicht auf der Längsachse/Mittelachse der Form und des Hohlraums endet. Das Dornelement kann also zwischen dem proximalen Endabschnitt und dem distalen Endabschnitt abgewinkelt sein, wobei sich der Abwinkelungspunkt auf der Mittelachse der Form und des Hohlraums befindet. Das Dornelement kann zwischen dem proximalen Endabschnitt und dem distalen Endabschnitt auch eine Biegung aufweisen. Das Dornelement kann zwischen dem proximalen Endabschnitt und dem distalen Endabschnitt auch mehrfach abgewinkelt und/oder gebogen sein.

In der Vorrichtung kann des weiteren im Hohlraum zumindest ein Kabelelement zur Signalübertragung so angeordnet sein, dass die Enden des zumindest einen Kabelelementes jeweils in den Gegenlagern verankert sind.

In der Vorrichtung können sämtliche Zugdrähte, Schlauchelemente oder Dornelemente und Kabelelemente parallel zu der Längserstreckung des Hohlraums verlaufen.

In der Vorrichtung können sämtliche Zugdrähte parallel zu der Längserstreckung des Hohlraums verlaufen, und die Schlauchelemente und Kabelelemente spiralartig im Hohlraum angeordnet sein.

In der Vorrichtung kann der zumindest eine Zugdraht eine Beschichtung aufweisen, die das in den Hohlraum einzufüllende aushärtende Material abstößt.

In der Vorrichtung kann die Form aus mehreren im Querschnitt jeweils ein Kreissegment bildenden Formteilen bestehen, die zueinander schiebbar sind, wobei im zusammengeschobenen Zustand der gesamte Innenraum der Formteile den Hohlraum ausbildet.

In der Vorrichtung kann die Form aus mehreren kreisabschnittartigen Schalen-Formteilen bestehen, von denen mindestens einer mindestens einen Zugangskanal für das einzufüllende aushärtende Material aufweist.

In der Vorrichtung kann die Form aus einem einzigen länglichen Hohlprofil bestehen, dessen Innenraum den Hohlraum ausbildet.

Zumindest eines der Gegenlager kann eine Haltekappe sein, in der der zumindest eine Zugdraht entnehmbar einhängbar ist, und die jeweilige Haltekappe kann an einer Matritze gestützt sein.

In der Vorrichtung kann der Hohlraum im Querschnitt zylindrisch oder polygonal sein.

Im erfindungsgemäßen Verfahren zum Herstellen eines länglichen Hohlprofilelements, das zumindest einen Zugdraht aufweist, werden folgende Schritte ausgeführt: Bereitstellen einer Form, die einen mit einem aushärtenden Material befüllbaren abgedichteten länglichen Hohlraum ausbildet, wobei der Hohlraum sich zwischen zwei den Hohlraum abdichtenden Gegenlagern erstreckt; Einlegen zumindest einen Zugdrahtes in die Form derart, dass am distalen Gegenlager ein Ende des zumindest einen Zugdrahtes verankert ist, wobei im Hohlraum der Zugdraht so verläuft, dass er die Hohlraumwand nicht berührt; Befüllen des Hohlraums mit einem aushärtenden Material; Aushärten des aushärtenden Materials; und Öffnen der Form.

Im Verfahren kann nach dem Öffnen der Form der zumindest einen Zugdraht derart gezogen werden, dass sich der Zugdraht von dem ihn umgebenden ausgehärteten Material löst und sich ein Bewegungskanal für den Zugdraht bildet. Das aushärtende Material kann ein Elastomer sein.

Das Elastomer kann Silikon, ein Naturkautschuk oder ein Silikonkautschuk sein. Das längliche Hohlprofilelement kann eine Abwinkelungseinheit für ein Endoskop sein.

Die Merkmale der Erfindung können geeignet kombiniert werden.

Nachstehend ist die Erfindung detailliert anhand von Beispielen erläutert.

### Kurzbeschreibung der Zeichnungen

Figur 1 zeigt ein Anwendungsbeispiel eines erfindungsgemäßen länglichen Hohlprofilelements, wobei Fig. 1A eine Seitenansicht zeigt, Fig. 1B eine Schnittansicht zeigt und Fig. 1C eine perspektivische Ansicht zeigt.
Figur 2 zeigt eine perspektivische Ansicht des Anwendungsbeispiels von Fig. 1 in gebogenem Zustand.
Figur 3 zeigt eine perspektivische Ansicht des Anwendungsbeispiels von Fig. 1, wobei das erfindungsgemäße längliche Hohlprofilelement separat gezeigt ist.
Figur 4 zeigt eine Vorrichtung zum Herstellen des erfindungsgemäßen länglichen Hohlprofilelements, wobei Fig. 1A eine perspektivische Explosionsdarstellung zeigt, Fig. 4B eine perspektivische Ansicht zeigt, Fig. 4C eine Seitenansicht zeigt und Fig. 4D eine Schnittansicht zeigt.

Nachstehend sind Ausführungsbeispiele der vorliegenden Erfindung beschrieben.

### Erstes Ausführungsbeispiel

Zunächst ist unter Bezugnahme auf die Zeichnungen ein erstes Ausführungsbeispiel erläutert.

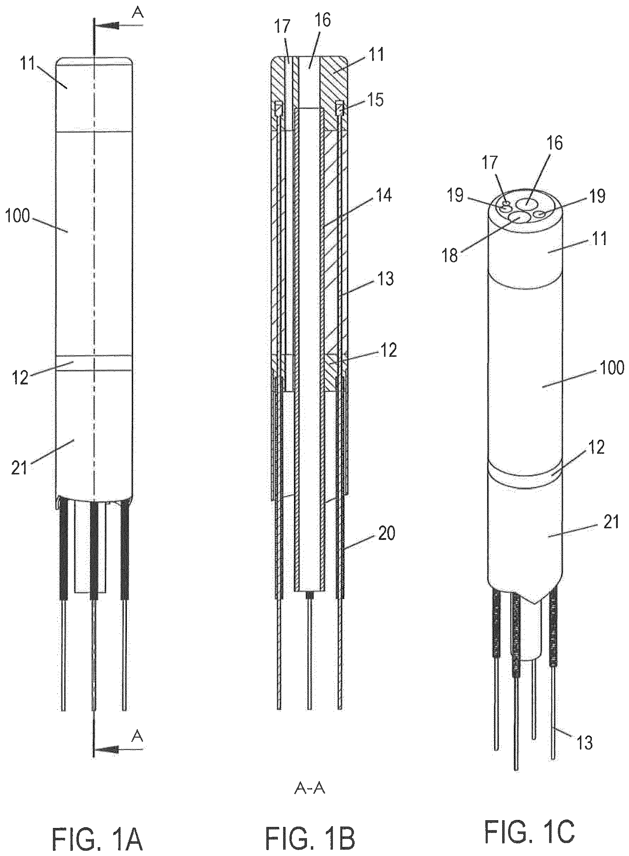

Figur 1 zeigt ein Anwendungsbeispiel eines erfindungsgemäßen länglichen Hohlprofilelements, wobei Fig. 1A eine Seitenansicht zeigt, Fig. 1B eine Schnittansicht entlang A-A in Fig. 1A zeigt und Fig. 1C eine perspektivische Ansicht zeigt.

Im ersten Ausführungsbeispiel ist die vorliegende Erfindung bei einem Mantelelement einer Abwinkelungseinheit (auch Deflectingabschnitt genannt) für ein Endoskop angewendet. Das Mantelelement ist hierbei ein längliches Hohlprofilelement im Sinne der Erfindung.

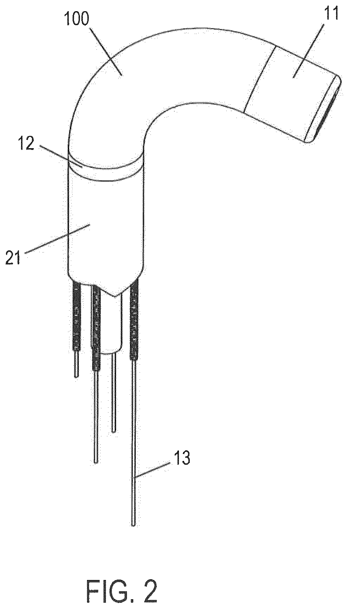

Eine solche Abwinkelungseinheit ist anhand der Figuren 1 und 2 nachstehend genauer erläutert.

Die Abwinkelungseinheit hat am distalen Ende einen Endoskopkopf 11, in welchem beispielsweise eine nicht gezeigte Kamera und nicht gezeigte LED's angeordnet sind. In proximaler Richtung der Abwinkelungseinheit ist an der proximalen Seite des Endoskopkopfes 11 ein längliches Hohlprofilelement 100 angeordnet, in dessen Inneren mehrere Zugseile 13 in Längsrichtung des länglichen Hohlprofilelements 100 laufen. Im vorliegenden Beispiel sind im länglichen Hohlprofilelement 100 vier Zugseile 13 vorgesehen. Es können auch drei, fünf oder mehr Zugseile 13 vorgesehen sein.

An der proximalen Seite des länglichen Hohlprofilelementes 100 ist ein Zwischenstück 12 angeordnet, das der Verankerung von Seilzug-Spiralhüllen 20 dient, in denen die Zugseile 13 proximal vom Zwischenstück 12 laufen. Das Zwischenstück 12 ist als ein Ringelement aufgebaut.

An der proximalen Seite des Zwischenstücks 12 ist ein Katheterschlauch 21 angeordnet, der zu einem Endoskopbedienteil führt. Der Katheterschlauch 21 umgibt die Seilzug-Spiralhüllen 20, in denen die Zugseile 13 verschiebbar laufen.

Die Abwinkelungseinheit erstreckt sich somit von dem Ringelement 12 an der proximalen Seite der Abwinkelungseinheit zu dem Endoskopkopf 11 an der distalen Seite der Abwinkelungseinheit.

Nachstehend sind die Elemente der Abwinkelungseinheit detaillierter erläutert.

Der Endoskopkopf 11 kann ein Kunststoffträger aus einem organischen polymeren Material sein, der z.B. durch Spritzgießen herstellbar ist. Beispielsweise besteht der Endoskopkopf 11 aus einem Thermoplast oder einem Duroplast. Insbesondere kann der Endoskopkopf 11 aus Polypropylen (PP), Acrylnitryl-Butadienstyrol (ABS), Polycarbonat (PC), Polyethylenterephtalat (PET), Polybutylenterephtalat (PBT), Polyamid (PA), Polyphenylensulfid (PPS), Polysulfon (PSU), Polyethersulfon (PES), Polyetherimid (PEI), etc hergestellt werden. Diese Angaben sind lediglich Beispiele und weitere Materialen können für den Endoskopkopf 11 verwendet werden.

An der distalen Stirnfläche besitzt der Endoskopkopf 11 distale Öffnungen 19 - 19. Diese Öffnungen können insbesondere eine distale Öffnung eines Arbeitskanals 16, eine distale Öffnung eines Spülkanals 17, ein Kamerafenster 18 und ein oder mehrere LED-Fenster 19 sein. Das Kamerafenster 18 dient einer Signaleingabe für eine nicht gezeigte Kamera und das LED-Fenster 19 dient einer Signalausgabe für einen nicht gezeigten LED-Chip.

Somit weist der Endoskopkopf 11 einen Arbeitskanal 16 und zumindest einen Spülkanal 17 auf. Im vorliegenden Beispiel weist der Endoskopkopf 11 einen Spülkanal 17 auf. Der Endoskopkopf 11 kann aber auch mehrere Spülkanäle haben. Genauer gesagt sind der Arbeitskanal 16 und der Spülkanal 17 im Endoskopkopf 11 in axialer Richtung parallel zur Mittelachse des Endoskopkopfes 11 vorgesehen. Wie in Fig. 1B und Fig. 1C gezeigt, ist der Spülkanal 17 radial außerhalb des Arbeitskanals 16 angeordnet, wobei die Erfindung nicht darauf beschränkt ist.

Beabstandet zur proximalen Endfläche besitzt der Endoskopkopf 11 an seiner Außenumfangsfläche jeweilige Einbauchungen für Zugseilverankerungskörper 15. Ein solcher Zugseilverankerungskörper 15 kann verschiedene Formen haben und kann ein Tonnennippel, ein Birnennippel, ein Kugelnippel etc. sein. Im vorliegenden Beispiel ist der Zugseilverankerungskörper 15 ein Tonnennippel. Jede der Einbauchungen hat eine derartige Größe, dass ein Zugseilverankerungskörper 15 darin Platz findet, und besitzt an ihrer proximalen Seite einen Abstützflächenbereich, an dem sich der Zugseilverankerungskörper 15 in proximaler Richtung abstützen kann. Der Abstützflächenbereich erstreckt sich somit horizontal d.h. parallel zur proximalen Endfläche des Endoskopkopfes 11. Die Einbauchungen sind an die Form des Zugseilverankerungskörpers 15 angepasst und sind im vorliegenden Beispiel zylinderartig gestaltet. Somit hat im vorliegenden Beispiel der Endoskopkopf 11 vier solche Abstützflächenbereiche. Jeder Abstützflächenbereich ist zentrisch mit einer zur proximalen Endfläche des Endoskopkopfes 11 laufenden rinnenartigen Einkerbung versehen, die bei am Abstützflächenbereich eingesetzten Zugseilverankerungskörper 15 das mit dem Zugseilverankerungskörper 15 verbundene Zugseil 13 aufnimmt. Der Durchmesser des Abstützflächenbereiches ist auf jeden Fall größer als der Durchmesser des Zugseils 13. Sollte z.B. ein quaderartiger Zugseilverankerungskörper angewendet werden und die Einbauchung daher eine quaderartige Form haben, ergibt sich ein viereckiger Abstützflächenbereich.

In jede seitliche Einbauchung am Endoskopkopf 11 ist ein Zugseilverankerungskörper 15 so eingesetzt, dass seine proximalen Seite am Abstützflächenbereich anliegt, so dass eine in proximaler Richtung wirkende Zugkraft vom Zugseilverankerungskörper 15 auf den Abstützflächenbereich und somit auf den Endoskopkopf 11 übertragen wird. Jeder Zugseilverankerungskörper 15 ist am distalen Ende eines Zugseils 13 in bekannter Weise fest angeordnet.

Im Endoskopkopf 11 verläuft entlang seiner Längsachse der Arbeitskanal 16 von der proximalen zu der distalen Richtung. An der proximalen Seite des Endoskopkopf 11 besitzt der Endoskopkopf 11 eine Durchmessererweiterung am Eingang für den Arbeitskanal 16. In diese Durchmessererweiterung ist ein Schlauchelement 14 eingesetzt. Das Schlauchelement 14 bildet an seiner Innenumfangsfläche einen Abschnitt des Arbeitskanals 16 aus, wie dies in Fig. 1B gezeigt ist. Das Schlauchelement 14 kann in den Endoskopkopf 11 eingeklebt oder eingepresst sein oder anderweitig befestigt sein.

Das erfindungsgemäße längliche Hohlprofilelement 100 ist ein schlauchartiges Gebilde, das den Arbeitskanal 16 radial umgibt. Im vorliegenden Beispiel ist umgibt das längliche Hohlprofilelement 100 den Arbeitskanal 16 so, dass das Schlauchelement 14 zwischen dem Arbeitskanal 16 und dem länglichen Hohlprofilelement 100 angeordnet ist. In einer Alternative kann das Schlauchelement 14 auch weggelassen werden. Dann bildet das längliche Hohlprofilelement 100 an der Innenumfangsseite somit den Arbeitskanal 16 direkt aus. Das längliche Hohlprofilelement 100 besteht aus einem Elastomer. Insbesondere kann Silikon, ein Naturkautschuk oder ein Silikonkautschuk etc. für das längliche Hohlprofilelement 100 verwendet werden.

An der distalen Seite liegt das längliche Hohlprofilelement 100 dicht am Endoskopkopf 11 an. An der proximalen Seite liegt das längliche Hohlprofilelement 100 dicht am Zwischenstück 12 an. Entlang seiner Längserstreckung sind im Inneren des länglichen Hohlprofilelements 100 parallel zur Längsachse des länglichen Hohlprofilelements 100 die Zugseile 13 angeordnet. Im länglichen Hohlprofilelement 100 sind daher zwischen dem Innenumfang und dem Außenumfang axiale durchgehende Öffnungen erfindungsgemäß geschaffen, in denen die Zugseile 13 laufen. Die axialen durchgehenden Öffnungen für die Zugseile 13 sind im länglichen Hohlprofilelement 100 - unter Betrachtung senkrecht zur Achse des länglichen Hohlprofilelements 100 - auf einem gedachten Kreis angeordnet, der näher zum Außenumfang als zum Innenumfang des länglichen Hohlprofilelements 100 beabstandet ist. Die Zugseile 13 sind somit zwischen dem Zwischenstück 12 und dem Endoskopkopf 11 im länglichen Hohlprofilelement 100 geschützt geführt. Im vorliegenden Beispiel sind vier axiale durchgehende Öffnungen für vier Zugseile 13 im länglichen Hohlprofilelement 100 vorgesehen. In der jeweiligen axialen durchgehenden Öffnung für die Zugseile 13 kann das entsprechende Zugseil 13 relativ zu der es umgebenden axialen durchgehenden Öffnung axial gezogen werden.

Das längliche Hohlprofilelement 100 besitzt im vorliegenden Beispiel einen sich axial erstreckenden parallel zu dem Arbeitskanal 16 und den vier axialen durchgehenden Öffnungen für die Zugseile 13 verlaufenden durchgehenden Kanal als Spülkanal 17. Der Spülkanal im länglichen Hohlprofilelement 100 ist zwischen dem Arbeitskanal 16 und dem gedachten Kreis, an dem die axialen durchgehenden Öffnungen für die Zugseile 13 angeordnet sind, im länglichen Hohlprofilelement 100 vorgesehen.

Das Zwischenstück 12 hat an der proximalen Seite Sacklöcher für die Verankerung der Seilzug-Spiralhüllen 20. Im Zwischenstück 12 sind die Seilzug-Spiralhüllen 20 von der proximalen Seite des Zwischenstücks 12 aus eingeführt. Vorzugsweise sind die Seilzug-Spiralhüllen 20 fest mit dem Zwischenstück 12 verbunden. Sie können z.B. eingeklebt sein. Der Durchmesser der Sacklöcher im Zwischenstück 12 kann auch so gewählt werden, dass die Seilzug-Spiralhüllen 20 darin eingepresst werden.

Das Zwischenstück 12 besitzt eine axiale zentrische Innenöffnung, durch die der Arbeitskanal läuft. Im vorliegenden Beispiel ist daher in der Innenöffnung des Zwischenstücks 12 das druckstabile Schlauchelement 14 für Arbeitskanal angeordnet.

Die zentrische Innenöffnung für das Schlauchelement 14 und eine seitliche Durchgangsöffnung für den Spülkanal 17 sind im Zwischenstück 12 in axialer Richtung parallel zur Mittelachse des Zwischenstücks 12 vorgesehen. Wie in Fig. 1B gezeigt, ist die seitliche Durchgangsöffnung für den Spülkanal 17 radial außerhalb der zentrischen Innenöffnung angeordnet. Die Anordnung der seitlichen Durchgangsöffnung für den Spülkanal 17 und der zentrischen Innenöffnung für das Schlauchelement 14 des Arbeitskanals 16 entsprechen der Anordnung des Spülkanal 17 und des Arbeitskanals 16 im Endoskopkopf 11.

Figur 2 zeigt eine perspektivische Ansicht des Anwendungsbeispiels von Fig. 1 in gebogenem Zustand.

Der Endoskopkopf 11 kann anhand einer Zugbewegung an den Zugseilen 13 durch ein nicht gezeigtes Steuerelement in eine beliebige Richtung in bekannter Weise geschwenkt werden. Wie dies in der Zeichnung gezeigt ist, ist hierbei das rechte Zugseil 13 stärker als das vordere und das hintere Zugseil in die proximale Richtung gezogen worden, so dass der Endoskopkopf 11 sich nach rechts neigt und das an ihm verankerte linke Zugseil 13 in die distale Richtung zieht.

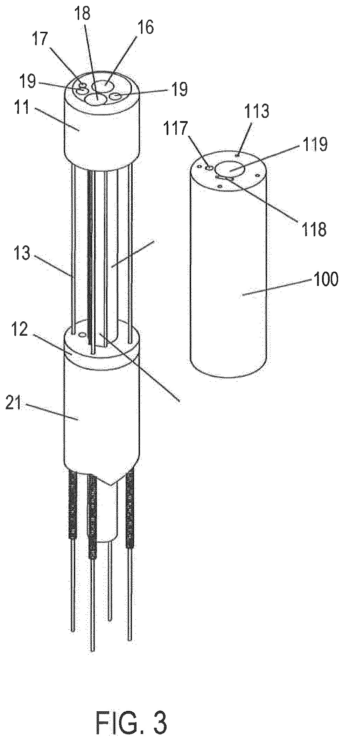

Figur 3 zeigt eine perspektivische Ansicht des Anwendungsbeispiels von Fig. 1, wobei das erfindungsgemäße längliche Hohlprofilelement separat gezeigt ist.

Insbesondere zeigt Fig. 3 das erste Ausführungsbeispiel, bei dem die Abwinkelungseinheit ohne das längliche Hohlprofilelement und das längliche Hohlprofilelement 100 separat gezeigt sind. Anders ausgedrückt ist das längliche Hohlprofilelement 100 der besseren Übersichtlichkeit wegen nicht in Einbaulage gezeigt.

Die Darstellung der Abwinkelungseinheit entspricht der Darstellung von Fig. 1C, wobei das Innere im Bereich des länglichen Hohlprofilelements 100 gezeigt ist. Insbesondere ist auch ein Kabel 22 für die Kamera gezeigt. Das Kabel 22 verläuft ähnlich wie der Spülkanal 17 axial im länglichen Hohlprofilelement 100 parallel zu dem Arbeitskanal 16 und den vier axialen durchgehenden Öffnungen für die Zugseile 13. Im vorliegenden Beispiel hat das Kabel 22 eine flache im Querschnitt rechteckige Form, an die der Kanal 118 im länglichen Hohlprofilelement 100 angepasst ist. Der Kanal 118 im länglichen Hohlprofilelement 100 ist zwischen dem Arbeitskanal 16 und dem gedachten Kreis, an dem die axialen durchgehenden Öffnungen für die Zugseile 13 angeordnet sind, im länglichen Hohlprofilelement 100 vorgesehen.

Das das längliche Hohlprofilelement 100 weist an der proximalen und distalen Seite (nur die distale Seite ist in Fig. 3 erkennbar) vier Öffnungen 113 für die Zugseile 13, eine Öffnung 117 für den Spülkanal 17, eine Öffnung 118 für das Kabel 22 der Kamera und eine Öffnung 118 für den Arbeitskanal 18 auf.

### Herstellung des länglichen Hohlprofilelements

Nachstehend ist die Herstellung des länglichen Hohlprofilelements 100 beschrieben.

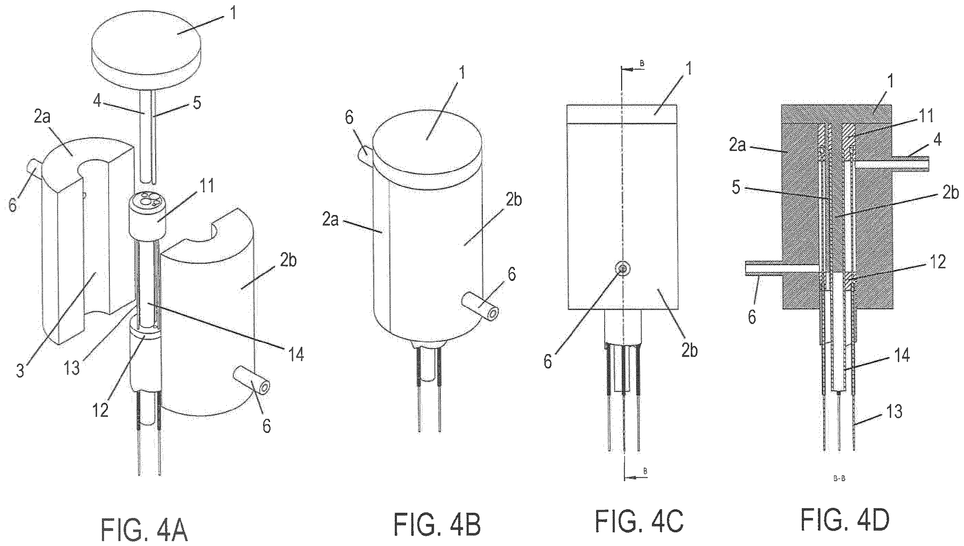

Figur 4 zeigt eine Vorrichtung zum Herstellen des erfindungsgemäßen länglichen Hohlprofilelements, wobei Fig. 1A eine perspektivische Explosionsdarstellung zeigt, Fig. 4B eine perspektivische Ansicht zeigt, Fig. 4C eine Seitenansicht zeigt und Fig. 4D eine Schnittansicht zeigt.

Die Abwinkelungseinheit wird in einem Zustand, bei dem sie mit Ausnahme des länglichen Hohlprofilelements 100 fertig montiert ist, bereitgehalten. Die Abwinkelungseinheit in einem Zustand ist nachstehend als Rohabwinkelungseinheit bezeichnet.

Eine Form, die im vorliegenden Beispiel aus zwei Halbformen 2a und 2b besteht, besitzt im Inneren eine zylindrische Erstreckung als die Gegenform für den Außenumfang des länglichen Hohlprofilelements 100. Der Außendurchmesser des Endoskopkopfes 11, des länglichen Hohlprofilelementes 100 und des Zwischenstücks 12 sind gleich, und der Innendurchmesser der zylindrischen Erstreckung ist an diesen Außendurchmesser des Endoskopkopfes 11, des länglichen Hohlprofilelementes 100 und des Zwischenstücks 12 angepasst.

Die Halbformen 2a und 2b sind im Querschnitt jeweils halbkreisartig und bilden zusammen einen Hohlzylinder, dessen Innenraum einen Hohlraum 3 für die Herstellung des länglichen Hohlprofilelementes 100 ausbildet. Der Hohlraum 3 ist zylinderartig. Seine Innenform ist an die Außenform des länglichen Hohlprofilelementes 100 angepasst.

Die Halbformen 2a und 2b sind zueinander hin und voneinander weg schiebbar. Wenn sie zusammengeschoben sind, ist der Hohlraum 3 am Umfang geschlossen.

Anders ausgedrückt, besteht die Form aus mehreren im Querschnitt jeweils einen Halbkreis bildenden Formteilen 2a und 2b, die zueinander schiebbar sind, wobei im zusammengeschobenen Zustand der gesamte Innenraum der Formteile den Hohlraum 3 ausbildet.

In den Hohlraum 3 wird die Abwinkelungseinheit in dem Zustand, bei dem sie mit Ausnahme des länglichen Hohlprofilelements 100 fertig montiert ist d.h. die Rohabwinkelungseinheit, so eingelegt, dass die distale Stirnfläche des Endoskopkopfes 11 an der distalen Stirnfläche der Formteile 2a und 2b fluchtet, wie dies in Fig. 4D erkennbar ist. In dem im Hohlraum 3 eingelegten Zustand bilden der Endoskopkopf 11 und das Zwischenstück 12 der Abwinkelungseinheit jeweils Gegenlager, die die darin laufenden Zugseile 13 spannen und zwischen denen das längliche Hohlprofilelement 100 hergestellt werden soll. Der Endoskopkopf 11 bildet ein erstes Gegenlager und das Zwischenstück 12 bildet ein zweites Gegenlager. Jedes Gegenlager stellt eine Haltekappe dar, die dafür sorgt, dass die Zugseile 13 gespannt gehalten werden können.

In dem Bereich des Hohlraums 3 der Formteile 2a und 2b, im eingelegten Zustand des Endoskopkopfes 11 und des Zwischenstückes 12 sich zwischen dem Endoskopkopf 11 und dem Zwischenstück 12 erstreckt, ist in jedem Formteil 2a und 2b eine radiale Öffnung so vorgesehen, dass sie radial die Wand des Formteils 2a und 2b durchdringt. An der Außenseite des Formteils 2a und 2b setzt sich die Öffnung zu einem Zugangsrohr fort. Die Öffnung und das Zugangsrohr bilden jeweils ein Zugangskanalelement 6 zur Befüllung des Hohlraums 3 mit dem einzufüllenden aushärtenden Material für das längliche Hohlprofilelement 100. Im vorliegenden Beispiel befindet sich das Zugangskanalelement 6 des Formteils 2a weiter an der distalen Seite und das Zugangskanalelement 6 des Formteils 2b befindet sich weiter an der proximalen Seite.

An die distale Stirnfläche des Endoskopkopfes 11 und an die distale Stirnfläche der Formteile 2a und 2b wird eine scheibenartige Matrize 1 gesetzt. Die scheibenartige Matrize 1 besitzt an ihrer proximalen Seite einen Stabilisierungskörper 4 für das Schlauchelement 14 für den Arbeitskanal und ein Formelement 5 für den Spülkanal. Der einen Stabilisierungskörper 4 und das Formelement 5 erstrecken sich senkrecht zur scheibenartigen Matrize 1. Genauer gesagt ist der Stabilisierungskörper 4 auf der Mittelachse der scheibenartigen Matrize 1 angeordnet und das Formelement 5 ist benachbart und parallel zum Stabilisierungskörper 4 angeordnet. Die Lage zwischen Stabilisierungskörper 4 und Formelement 5 ist so gewählt, dass das Formelement 5 den Spülkanal ausbildet, während der Stabilisierungskörper 4 den Arbeitskanal ausbildet oder das Schlauchelement 14 zur Ausbildung des Arbeitskanals von innen stützt.

Wenn die Formhälften 2a und 2b bei eingelegter Rohabwinkelungseinheit geschlossen sind, wird die scheibenartige Matrize 1 von der distalen Seite her auf die distalen Stirnflächen der Formhälften 2a und 2b so aufgeschoben, dass der Stabilisierungskörper 4 in das Schlauchelement 14 einfährt, wie dies den Figuren 4A bis 4D entnehmbar ist. Somit ist die Form geschlossen. In diesem Zustand verläuft im Hohlraum 3 der Zugdraht 13 so, dass er die Hohlraumwand nicht berührt.

Nun wird das das längliche Hohlprofilelement 100 ausbildende Elastomermaterial in die Form eingegossen. Insbesondere wird das Elastomermaterial durch eines der beiden Zugangskanalelemente 6 eingegossen, während das Zugangskanalelement 6 als Luftabgabeöffnung dient, durch die die im Hohlraum 3 befindliche durch das eingegossene Elastomermaterial verdrängte Luft entweichen kann. Wenn der Innenraum der Form mit Elastomermaterial gefüllt, z.B. wenn das Elastomermaterial aus dem als Luftabgabeöffnung dienenden Zugangskanalelement 6 austritt oder wenn ein Sensor am innen befindlichen Eingang des als Luftabgabeöffnung dienenden Zugangskanalelement 6 ein Vorhandensein von Elastomermaterial anzeigt, werden die Zugangskanalelemente 6 geschlossen.

Das Elastomermaterial härtet aus. Daraufhin wird die Form geöffnet und die Abwinkelungseinheit wird entnommen. Am Außenumfang des länglichen Hohlprofilelements 100 noch vorhandenes Elastomermaterial, das dem Innenraum der Zugangskanalelemente 6 entspricht, wird abgeschnitten. Alternativ kann an jeder Form 2a, 2b am Innenumfang des Hohlraums 3 am Mündungsbereich der Zugangskanalelemente 6 zum Hohlraum 3 ein Schieber angeordnet sein, der nach Einfüllen des Elastomermaterial das jeweilige Zugangskanalelement 6 versperrt.

Nach dem Öffnen der Form 2a, 2b werden die Zugseile 13 derart gezogen, dass sich die Zugseile 13 von dem sie umgebenden Elastomermaterial lösen. Dadurch bildet sich ein Bewegungskanal für die Zugseile 13. Vorzugsweise sind die Zugseile 13 mit einer Beschichtung versehen, die das Elastomermaterial absto-ßen.

Somit wird eine Abwinkelungseinheit erzeugt, die ein längliches Hohlprofilelement 100 hat, in welchem Zugseile 13 geführt werden und ein Arbeitskanal verläuft. Das längliche Hohlprofilelement 100 ist ein Mantelelement der Abwinkelungseinheit.

### Alternativen

Im ersten Beispiel sind eine Kamera und LED-Elemente im Endoskopkopf 11 angeordnet. Alternativ kann anstelle der LED-Elemente eine Ultraschallemittiereinrichtung angeordnet sein, und anstelle der Kamera kann ein Akustiksensor angeordnet sein.

Im ersten Beispiel bildet das Schlauchelement 14 an seiner Innenumfangsfläche einen Abschnitt des Arbeitskanals 16 aus. Der distale Endabschnitt des Arbeitskanals 16 ist dabei im Endoskopkopf 11 ohne das Schlauchelement 14 als Loch im Endoskopkopf 11 ausgebildet. Alternativ kann das Schlauchelement 14 sich bis zu der distalen Endseite des Endoskopkopfes 11 erstrecken.

In einer anderen Alternative kann auf das Schlauchelement 14 in dem Bereich zwischen Endoskopkopf 11 und Zwischenstück 12 verzichtet werden. In diesem Fall kann das Schlauchelement 14 im Zwischenstück 12 enden.

In diesem Fall kann die Konstruktion so gewählt werden, dass der Arbeitskanal 16 nur durch ein Dornelement 4 gebildet wird. Das Dornelement 4 entspricht dem Stabilisierungskörper 4 aus Fig. 4A. Der Durchmesser des Dornelements 4 ist an den auszubildenden Enddurchmesser des Arbeitskanals angepasst.

Die Anordnung der Kanäle im Endoskopkopf 11 ist nicht einschränkend und kann je nach Bedarf abgewandelt werden.

Im Beispiel von Fig. 4 besteht die Form aus mehreren im Querschnitt jeweils einen Halbkreis bildenden Formteilen 2a und 2b, die zueinander schiebbar sind, wobei im zusammengeschobenen Zustand der gesamte Innenraum der Formteile den Hohlraum 3 ausbildet. Die Form kann auch aus drei oder mehr im Querschnitt jeweils ein Kreissegment bildenden Formteilen bestehen, die zueinander schiebbar sind, wobei im zusammengeschobenen Zustand der gesamte Innenraum der Formteile den Hohlraum 3 ausbildet. In einer weiteren Alternative besteht die Form aus einem einzigen länglichen Hohlprofil, dessen Innenraum den Hohlraum 3 ausbildet.

Nicht jedes Formteil muss einen Zugangskanal besitzen. Es ist ausreichend, dass die Form, wenn sie aus mehreren kreisabschnittartigen Schalen-Formteilen besteht, mindestens ein Formteil mit einem Zugangskanal für das einzufüllende aushärtende Material aufweist.

Im ersten Beispiel erstreckt sich das Dornelement 4 senkrecht von der Matritze 1, die quasi einen Deckel der Form 2a, 2b bildet. Somit liegt im ersten Beispiel das Dornelement 4 auf der Mittelachse der Form 2a, 2b. Dadurch wird ein gerader Arbeitskanal 16 ausgebildet, der in der Abwinkelungseinheit zentrisch verläuft. Alternativ kann das Dornelement 4 einen proximalen Endabschnitt, der sich auf der Mittelachse der Form 2a, 2b erstreckt, und einen distalen Endabschnitt haben, der unter einem Winkel zur der Mittelachse der Form 2a, 2b verläuft und aussermittig zur Mittelachse der Form 2a, 2b an der Matritze 1 befestigt ist.

Das Dornelement 4 kann somit zwischen dem proximalen Endabschnitt und dem distalen Endabschnitt abgewinkelt sein, wobei sich der Abwinkelungspunkt auf der Mittelachse der Form 2a, 2b und des Hohlraums 3 befindet. Das Dornelement 4 kann zwischen dem proximalen Endabschnitt und dem distalen Endabschnitt auch eine Biegung mit einem Radius aufweisen. Das Dornelement 4 kann sogar zwischen dem proximalen Endabschnitt und dem distalen Endabschnitt mehrfach abgewinkelt und/oder gebogen sein oder schlangenlinienförmig verlaufen. Da das Elastomermaterial auch nach dem Aushärten elastisch ist, ist ein Herausziehen des Dornelementes 4 aus dem ausgebildeten und ausgehärteten länglichen Hohlprofilelement 100 ohne Probleme möglich, selbst wenn das Dornelement 4 abgewinkelt, gebogen oder schlangenlinienförmig ist. Dadurch kann ein Arbeitskanal 16 geschaffen werden, der am distalen Ende nicht zentrisch sondern aussermittig an der distalen Fläche des Endoskopkopfes 11 endet. Dadurch steht für eine Kamera und ein Kamerafenster 18 mehr Platz zu Verfügung, die näher zur Mitte hin als im ersten Beispiel angeordnet werden können.

In ähnlicher Weise kann auch das Formelement 5 für den Spülkanal 17 bei Bedarf abgewinkelt, gebogen oder schlangenlinienförmig sein.

### Bezugszeichenliste

- 1: Matrize
- 2a, 2b: Formkörper
- 3: Hohlraum
- 4: Stabilisierungskörper; Dornelement
- 5: Formelement für Spülkanal
- 6: Zugangskanalelement
- 11: Endoskopkopf; erstes Gegenlager
- 12: Zwischenstück; zweites Gegenlager
- 13: Zugseil
- 14: Schlauchelement für Arbeitskanal
- 15: Zugseilverankerungskörper
- 16: Arbeitskanal
- 17: Spülkanal
- 18: Kamerafenster
- 19: LED-Fenster
- 20: Spiralhülle für Zugseil
- 21: Katheterschlauch
- 22: Kabel für Kamera
- 100: längliches Hohlprofilelement
- 113: Öffnungen für die Zugseile 13
- 117: Öffnung für Spülkanal 17
- 118: Öffnung für Kabel 22
- 119: Öffnung für den Arbeitskanal 16

## Patentansprüche

1. Vorrichtung zum Herstellen eines länglichen Hohlprofilelements (100) mit zumindest einer axialen Öffnung, in der ein Zugdraht (13) axial relativ zum Hohlprofilelement (100) ziehbar angeordnet ist, wobei der Zugdraht (13) zur Übertragung einer Schwenkbewegung eines Steuerelements zu einer Abwinkelungseinheit dient, mit:
zwei an entgegengesetzten Seiten der Vorrichtung angeordneten Gegenlagern (11, 12), die den Längsenden des länglichen Hohlprofilelements entsprechen, wobei am distalen Gegenlager (11) ein Ende des zumindest einen Zugdrahtes (13) verankert ist, und
einer Form (2a, 2b), die einen mit einem aushärtenden Material, das das Hohlprofilelement (100) ausbildet, befüllbaren abgedichteten länglichen Hohlraum (3) ausbildet, wobei der Hohlraum (3) sich zwischen den beiden Gegenlagern (11, 12) erstreckt und im Hohlraum (3) der Zugdraht (13) so verläuft, dass er die Hohlraumwand nicht berührt,
wobei in die Form (2a, 2b) die beiden Gegenlager (11, 12) eingelegt sind, und
wobei am Ende der Herstellung der zumindest eine Zugdraht (13) im ausgehärteten Material axial relativ zum Hohlprofilelement (100) ziehbar angeordnet bleibt.

2. Vorrichtung gemäß Anspruch 1, wobei
die Gegenlager (11, 12) so beabstandet sind, dass der Zugdraht (13) gespannt ist.

3. Vorrichtung gemäß einem der Ansprüche 1 oder 2, wobei
außer dem zumindest einen Zugdraht (13) im Hohlraum (3) zumindest ein Schlauchelement (14) zur Ausbildung eines Arbeitskanals so angeordnet ist, dass die Enden des zumindest einen Schlauchelementes (14) jeweils in den Gegenlagern (11, 12) verankert sind.

4. Vorrichtung gemäß einem der Ansprüche 1 oder 2, wobei
außer dem zumindest einen Zugdraht (13) im Hohlraum (3) zumindest ein entnehmbares Dornelement (4) zur Ausbildung eines Arbeitskanals so angeordnet ist, dass die Enden des zumindest einen Dornelementes jeweils in den Gegenlagern sitzen.

5. Vorrichtung gemäß Anspruch 4, wobei
das Dornelement (4) am distalen Ende einen außermittigen Endabschnitt aufweist.

6. Vorrichtung gemäß einem der Ansprüche 1 bis 5, wobei
des Weiteren im Hohlraum (3) zumindest ein Kabelelement zur Signalübertragung so angeordnet ist, dass die Enden des zumindest einen Kabelelementes jeweils in den Gegenlagern (11, 12) verankert sind.

7. Vorrichtung gemäß einem der Ansprüche 1 bis 6, wobei
sämtliche Zugdrähte (13), Schlauchelemente (14) oder Dornelemente (4) und Kabelelemente parallel zu der Längserstreckung des Hohlraums (3) verlaufen.

8. Vorrichtung gemäß einem der Ansprüche 1 bis 6, wobei
sämtliche Zugdrähte (13) und die Schlauchelemente parallel zu der Längserstreckung des Hohlraums (3) verlaufen, und Kabelelemente spiralartig im Hohlraum (3) angeordnet sind.

9. Vorrichtung gemäß einem der Ansprüche 1 bis 8, wobei
der zumindest eine Zugdraht (13) eine Beschichtung aufweist, die das in den Hohlraum (3) einzufüllende aushärtende Material abstößt.

10. Vorrichtung gemäß einem der Ansprüche 1 bis 9, wobei
die Form aus mehreren im Querschnitt jeweils ein Kreissegment bildenden Formteilen (2a, 2b) besteht, die zueinander schiebbar sind, wobei im zusammengeschobenen Zustand der gesamte Innenraum der Formteile (2a, 2b) den Hohlraum (3) ausbildet.

11. Vorrichtung gemäß Anspruch 10, wobei
die Form aus mehreren kreisabschnittartigen Schalen-Formteilen (2a, 2b) besteht, von denen mindestens einer mindestens einen Zugangskanal (6) für das einzufüllende aushärtende Material aufweist.

12. Vorrichtung gemäß einem der Ansprüche 1 bis 9, wobei
die Form aus einem einzigen länglichen Hohlprofil besteht, dessen Innenraum den Hohlraum (3) ausbildet.

13. Vorrichtung gemäß einem der Ansprüche 1 bis 12, wobei
zumindest eines des Gegenlager (11) eine Haltekappe ist, in der der zumindest eine Zugdraht (13) entnehmbar einhängbar ist, und
die jeweilige Haltekappe an einer Matritze gestützt ist.

14. Vorrichtung gemäß einem der Ansprüche 1 bis 13, wobei
der Hohlraum (3) im Querschnitt zylindrisch oder polygonal ist.

15. Verfahren zum Herstellen eines länglichen Hohlprofilelements mit zumindest einer axialen Öffnung, in die ein Zugdraht (13) axial relativ zum Hohlprofilelement (100) ziehbar angeordnet ist, wobei der Zugdraht (13) zur Übertragung einer Schwenkbewegung eines Steuerelements zu einer Abwinkelungseinheit dient, mit den folgenden Schritten:
Bereitstellen einer Form (2a, 2b), die einen mit einem aushärtenden Material befüllbaren abgedichteten länglichen Hohlraum (3) ausbildet, wobei der Hohlraum (3) sich zwischen zwei den Hohlraum (3) abdichtenden Gegenlagern (11, 12) erstreckt;
Einlegen der beiden Gegenlager (11, 12) mit zumindest einem Zugdraht (13) in die Form (2a, 2b) derart, dass am distalen Gegenlager (11) ein Ende des zumindest einen Zugdrahtes (13) verankert ist, wobei im Hohlraum (3) der Zugdraht (13) so verläuft, dass er die Hohlraumwand nicht berührt;
Befüllen des Hohlraums (3) mit einem aushärtenden Material zur Ausbildung des Hohlprofilelements (100);
Aushärten des aushärtenden Materials; und
Öffnen der Form (2a, 2b),
wobei am Ende des Herstellverfahrens der zumindest ein Zugdraht (13) im ausgehärteten Material axial relativ zum Hohlprofilelement (100) ziehbar angeordnet bleibt.

16. Verfahren gemäß Anspruch 15, mit dem folgenden Schritt:
nach dem Öffnen der Form (2a, 2b):
Ziehen des zumindest einen Zugdrahtes (13) derart, dass sich der Zugdraht (13) von dem ihn umgebenden ausgehärteten Material löst und sich ein Bewegungskanal für den Zugdraht (13) bildet.

17. Verfahren gemäß einem der Ansprüche 15 oder 16, wobei das aushärtende Material ein Elastomer ist.

18. Verfahren gemäß Anspruch 17, wobei
das Elastomer Silikon, ein Naturkautschuk oder ein Silikonkautschuk ist.

## Claims

1. Device for producing an elongate hollow profile element (100) with at least one axial opening in which a pulling wire (13) is arranged to be axially pullable relative to the hollow profile element (100), wherein the pulling wire (13) serves to transmit a pivoting movement of a control element to a bending unit, with:
two counter-bearings (11, 12) arranged at opposite sides of the device and corresponding to the long ends of the elongate hollow profile element, wherein one end of the at least one pulling wire (13) is anchored to the distal counter-bearing (11), and
a mould (2a, 2b) forming a sealed elongate cavity (3) that can be filled with a hardening material that forms the hollow profile element (100), wherein the cavity (3) extends between the two counter-bearings (11, 12), and the pulling wire (13) extends in the cavity (3) such that it does not touch the cavity wall,
wherein the two counter-bearings (11, 12) are inserted into the mould (2a, 2b), and
wherein, at the end of production, the at least one pulling wire (13) remains arranged in the hardened material, axially pullable relative to the hollow profile element (100).

2. Device according to Claim 1, wherein the counter-bearings (11, 12) are spaced apart such that the pulling wire (13) is tensioned.

3. Device according to either of Claims 1 and 2, wherein, besides the at least one pulling wire (13), at least one tube element (14) for forming a working channel is arranged in the cavity (3) such that the ends of the at least one tube element (14) are anchored respectively in the counter-bearings (11, 12) .

4. Device according to either of Claims 1 and 2, wherein, besides the at least one pulling wire (13), at least one removable mandrel element (4) for forming a working channel is arranged in the cavity (3) such that the ends of the at least one mandrel element sit respectively in the counter-bearings.

5. Device according to Claim 4, wherein the mandrel element (4) has, at the distal end, an eccentric end portion.

6. Device according to one of Claims 1 to 5, wherein furthermore at least one cable element for signal transmission is arranged in the cavity (3) such that the ends of the at least one cable element are anchored respectively in the counter-bearings (11, 12) .

7. Device according to one of Claims 1 to 6, wherein all pulling wires (13), tube elements (14) or mandrel elements (4) and cable elements run parallel to the longitudinal extent of the cavity (3).

8. Device according to one of Claims 1 to 6, wherein all pulling wires (13) and the tube elements run parallel to the longitudinal extent of the cavity (3), and cable elements are arranged in a spiral shape in the cavity (3).

9. Device according to one of Claims 1 to 8, wherein the at least one pulling wire (13) has a coating which repels the hardening material to be introduced into the cavity (3).

10. Device according to one of Claims 1 to 9, wherein the mould consists of several mould parts (2a, 2b) which in cross section each form a circular segment and which can be pushed towards each other, wherein, in the pushed-together state, the whole interior of the mould parts (2a, 2b) forms the cavity (3).

11. Device according to Claim 10, wherein the mould consists of several shell-like mould parts (2a, 2b) like a segment of a circle, at least one of which has at least one access channel (6) for the hardening material that is to be introduced.

12. Device according to one of Claims 1 to 9, wherein the mould consists of a single elongate hollow profile, of which the interior forms the cavity (3).

13. Device according to one of Claims 1 to 12, wherein at least one of the counter-bearings (11) is a retaining cap in which the at least one pulling wire (13) is removably insertable, and the respective retaining cap is supported on a die.

14. Device according to one of Claims 1 to 13, wherein the cavity (3) is cylindrical or polygonal in cross section.

15. Method for producing an elongate hollow profile element with at least one axial opening in which a pulling wire (13) is arranged to be axially pullable relative to the hollow profile element (100), wherein the pulling wire (13) serves to transmit a pivoting movement of a control element to a bending unit, said method having the following steps:
making available a mould (2a, 2b) forming a sealed elongate cavity (3) that can be filled with a hardening material, wherein the cavity (3) extends between two counter-bearings (11, 12) that seal the cavity (3);
inserting the two counter-bearings (11, 12) with at least one pulling wire (13) into the mould (2a, 2b) in such a way that one end of the at least one pulling wire (13) is anchored to the distal counter-bearing (11), wherein the pulling wire (13) extends in the cavity (3) such that it does not touch the cavity wall;
filling the cavity (3) with a hardening material for forming the hollow profile element (100) ;
hardening the hardening material; and
opening the mould (2a, 2b),
wherein, at the end of the production method, the at least one pulling wire (13) remains arranged in the hardened material, axially pullable relative to the hollow profile element (100).

16. Method according to Claim 15, with the following step:
after opening the mould (2a, 2b):
pulling the at least one pulling wire (13) in such a way that the pulling wire (13) detaches from the hardened material surrounding it and a movement channel for the pulling wire (13) forms.

17. Method according to either of Claims 15 and 16, wherein the hardening material is an elastomer.

18. Method according to Claim 17, wherein the elastomer is silicone, a natural rubber or a silicone rubber.

## Revendications

1. Dispositif de fabrication d'un élément profilé creux allongé (100) pourvu d'au moins une ouverture axiale dans laquelle un fil de traction (13) est disposé de manière à pouvoir être tiré axialement par rapport à l'élément profilé creux (100), le fil de traction (13) servant à transmettre un mouvement de pivotement d'un élément de commande à une unité de pliage, le dispositif comprenant :
deux butées (11, 12) qui sont disposées sur des côtés opposés du dispositif et qui correspondent aux extrémités longitudinales de l'élément profilé creux allongé, une extrémité de l'au moins un fil de traction (13) étant ancrée au niveau de la butée distale (11), et
un moule (2a, 2b) qui forme une cavité allongée étanche (3) pouvant être remplie d'un matériau durcissant formant l'élément profilé creux (100), la cavité (3) s'étendant entre les deux butées (11, 12) et, dans la cavité (3), le fil de traction (13) s'étendant de manière à ne pas toucher la paroi de la cavité,
les deux butées (11, 12) étant insérées dans le moule (2a, 2b), et
à la fin de la fabrication, l'au moins un fil de traction (13) restant disposé dans le matériau durci de manière à pouvoir être tiré axialement par rapport à l'élément profilé creux (100).

2. Dispositif selon la revendication 1,
les butées (11, 12) étant espacées de manière à ce que le fil de traction (13) soit tendu.

3. Dispositif selon l'une des revendications 1 ou 2, dans lequel
outre l'au moins un fil de traction (13) dans la cavité (3), au moins un élément formant tuyau souple (14) destiné à former un canal de travail étant disposé de manière à ce que les extrémités de l'au moins un élément formant tuyau souple (14) soient ancrées dans les butées (11, 12).

4. Dispositif selon l'une des revendications 1 ou 2,
outre l'au moins un fil de traction (13) dans la cavité (3), au moins un élément formant mandrin amovible (4) destiné à former un canal de travail étant disposé de manière à ce que les extrémités de l'au moins un élément formant mandrin se situent chacune dans les butées.

5. Dispositif selon la revendication 4,
l'élément formant mandrin (4) comportant au niveau de l'extrémité distale une partie d'extrémité excentrée.

6. Dispositif selon l'une des revendications 1 à 5,
en outre, dans la cavité (3), au moins un élément formant câble destiné à la transmission de signaux étant disposé de manière à ce que les extrémités de l'au moins un élément formant câble soient chacune ancrées dans les butées (11, 12).

7. Dispositif selon l'une des revendications 1 à 6,
tous les fils de traction (13), les éléments formant tuyau souple (14) ou les éléments formant mandrin (4) et les éléments formant câble s'étendant parallèlement à l'extension longitudinale de la cavité (3).

8. Dispositif selon l'une des revendications 1 à 6,
tous les fils de traction (13) et les éléments formant tuyau souple s'étendant parallèlement à l'extension longitudinale de la cavité (3), et les éléments formant câble étant disposés en spirale dans la cavité (3).

9. Dispositif selon l'une des revendications 1 à 8,
l'au moins un fil de traction (13) comportant un revêtement qui repousse le matériau durcissant à introduire dans la cavité (3).

10. Dispositif selon l'une des revendications 1 à 9,
le moule comprenant une pluralité de parties de moule (2a, 2b) qui forment chacune en coupe transversale un segment de cercle et qui peuvent coulisser les unes vers les autres, tout l'espace intérieur des parties de moule (2a, 2b) formant, à l'état regroupé, la cavité (3).

11. Dispositif selon la revendication 10,
le moule comprenant plusieurs parties de moule (2a, 2b) en forme de coque de type partie de cercle dont au moins une comporte au moins un canal d'accès (6) destiné au matériau de durcissement à introduire.

12. Dispositif selon l'une des revendications 1 à 9,
le moule comprenant un profilé creux allongé unique dont l'espace intérieur forme la cavité (3).

13. Dispositif selon l'une des revendications 1 à 12,
au moins une des butées (11) étant un capuchon de retenue dans lequel l'au moins un fil de traction (13) peut être suspendu de manière amovible, et
le capuchon de retenue respectif étant en appui sur une filière.

14. Dispositif selon l'une des revendications 1 à 13,
la cavité (3) étant cylindrique ou polygonale en coupe transversale.

15. Procédé de fabrication d'un élément profilé creux allongé comportant au moins une ouverture axiale dans laquelle un fil de traction (13) est disposé de manière à pouvoir être tiré axialement par rapport à l'élément profilé creux (100), le fil de traction (13) servant à transmettre un mouvement de pivotement d'un élément de commande à une unité de pliage, le procédé comprenant les étapes suivantes :
prévoir un moule (2a, 2b) qui forme une cavité allongée étanche (3) pouvant être remplie avec un matériau durcissant, la cavité (3) s'étendant entre deux butées (11, 12) fermant la cavité (3) de manière étanche ;
insérer les deux butées (11, 12) avec au moins un fil de traction (13) dans le moule (2a, 2b) de manière à ce qu'une extrémité de l'au moins un fil de traction (13) soit ancrée sur la butée distale (11), le fil de traction (13) s'étendant dans la cavité (3) de manière à ne pas toucher la paroi de la cavité ;
remplir la cavité (3) avec un matériau durcissant pour former l'élément profilé creux (100) ;
faire durcir le matériau durcissant ; et
ouvrir le moule (2a, 2b),
à la fin du procédé de fabrication, l'au moins un fil de traction (13) restant disposé dans le matériau durci de manière à pouvoir être tiré axialement par rapport à l'élément profilé creux (100).

16. Procédé selon la revendication 15, comprenant l'étape suivante :
après l'ouverture du moule (2a, 2b) :
tirer l'au moins un fil de traction (13) de manière à ce que le fil de traction (13) se détache du matériau durci qui l'entoure et forme un canal de mouvement destiné au fil de traction (13).

17. Procédé selon l'une des revendications 15 ou 16,
le matériau durcissant étant un élastomère.

18. Procédé selon la revendication 17,
l'élastomère étant du silicone, un caoutchouc naturel ou un caoutchouc de silicone.
